# EUROPEAN PATENT SPECIFICATION

(11) **EP 3 151 744 B1**
(45) Date of publication and mention of the grant of the patent: **26.01.2022**
(21) Application number: 15726201.5
(22) Date of filing: 05.06.2015
(51) Int. Cl.: A61B 5/145, A61B 5/00, A61B 5/024, A61B 5/288

(54) **INTEGRATED ELECTRODE FOR SAMPLING OF LACTATE AND OTHER ANALYTES**
INTEGRIERTE ELEKTRODE ZUR PROBENAHME VON LAKTAT UND ANDEREN ANALYTEN
ÉLECTRODE INTÉGRÉE POUR ÉCHANTILLONNER LE LACTATE ET D'AUTRES ANALYTES

(30) Priority: 07.06.2014 EP 14171634
(43) Date of publication of application: 12.04.2017
(73) Proprietor: CINOX B.V., 9712 PB Groningen (NL)
(72) Inventor: CREMERS, Thomas Ivo Franciscus Hubert, 9747 AW Groningen (NL); VAN DEN BERG, Paul Pierre, 9712 PB Groningen (NL)
(74) Representative: EDP Patent Attorneys B.V.
(86) International application number: PCT/EP2015/062616
(87) International publication number: WO 2015/185745

(56) References cited:
- WO-A1-91/18549
- WO-A1-03/088837
- WO-A1-2009/142599
- CN-A- 101 642 380
- JP-A- 2012 179 271
- JP-A- 2013 066 531
- US-A- 4 658 825
- US-A- 5 640 954
- US-A1- 2005 208 648
- US-B1- 6 298 253

## Description

### FIELD OF THE INVENTION

The invention relates to a biosensor for at least partial insertion in the fetal scalp as well as to a sensing system comprising such biosensor.

### BACKGROUND OF THE INVENTION

Fetal sensors are known in the art. US5727547, for instance, describes a fetal pulse oximeter sensor with a securing mechanism for providing tension to a hook or other securing mechanism penetrating the fetal scalp. In one embodiment, a sensor can be secured to the presenting part of the fetus by means of a suture or a hook which penetrates the fetus' scalp. If a suture is used, the ends of the suture are wrapped around a securing member, such as a bar, attached to the back of the sensor. By tying the suture ends around this securing member, the sensor is pressed up against the fetus' scalp and retained in place by virtue of tension provided in the suture between the securing member and the portion of the suture penetrating the fetus' scalp.

US4658825A describes a combined spiral EKG electrode and pH probe for use in monitoring a fetus during labor. The combined probe includes a spiral-shaped needle which acts as an EKG electrode and also houses a fiberoptic pH probe. Optical fibers and wires from the needle as well as from a reference electrode on the probe assembly are passed back through the cervix and vagina of a woman in order to provide simultaneous information as to both EKG and pH of the fetus during labor.

WO2009142599A1 describes a diagnostic device for the monitoring of markers of global metabolomic status in the tissue of a fetus during birth, comprising a probe containing an inlet tube, an outlet tube, and a membrane portion, said membrane portion allowing the passage of markers of global metabolomic status from the tissue, said probe containing one or more anchoring means at the distal end of the probe in close proximity to the membrane portion. The device can be attached to the presenting part of the fetus (usually the head) through the birth-canal of the pregnant woman. The probe, which is a part of a disposable device, allows certain metabolites to perfuse from the tissues through the membrane of the probe. The metabolites are then transported by the microdialysis fluid to an analysis unit. The analysis performed on the fluid determines the concentration of certain metabolites such as lactate or pH. CTG functionality is be added to the disposable device for additional monitoring of the status of the fetus.

US5640954A describes a method and an apparatus for continuously monitoring the concentration of a metabolite, such as glucose or lactic acid, in biological tissue, in which a perfusion fluid is fed to a microdialysis probe implanted in the subcutaneous tissue and removed therefrom as dialysate after enrichment with the metabolites contained in the lymph. An enzyme is added to the dialysate and/or perfusion fluid, and the concentration of the metabolite in the dialysate is determined under the selective effect of the enzyme at a measuring point which is positioned ex vivo, using an electrochemical sensor. The enzyme is added to the dialysate in the form of a continuous enzyme solution flow upstream of the measuring point and in dependence of the throughput of the microdialysis probe.

### SUMMARY OF THE INVENTION

Monitoring of fetal functioning during labor is crucial in order to determine interventions. CTG (cardiotocography) electrode and STAN (ST waveform analysis of fetal electrocardiogram (ECG) for intrapartum surveillance) electrodes monitor heart function of the fetus. These electrodes are screwed in the scalp of the fetus for fixation and monitor fetal cardiac function.

Cardiac function, however, is not a good predictor for actual functioning of the fetus. To this end scalp blood samples are taken and analyzed e.g. for lactate levels (MBA, micro blood analysis). Lactate levels are known to be the right parameter for adequate monitoring and intervention decisions. However, taking blood samples for the scalp of the fetus is challenging. To this end, local continuous sampling techniques are warranted for adequate monitoring of a fetus during labor.

In the current application we describe the geometry and functioning of a microdialysis application that is incorporated into a (regular) fetal scalp electrode, like a fetal scalp electrode configured for CTG analysis and/or STAN analysis. To this end, a biosensor is provided that may retrieve an analyte from a body tissue or liquid, which analyte may be measured. The analyte can be measured quantitatively or qualitatively. The term analysis may refer to one or more qualitative and quantitative analysis. The term analyte may also refer to a plurality of analytes. Further, the term analysis may also include an analysis wherein no analyte is detected at all, for instance when not present or when below the detection level.

In general, microdialysis is a minimally-invasive sampling technique that is e.g. used for continuous measurement of free or bound analyte concentrations in the extracellular fluid of virtually any tissue. For the procedure to be carried out, small amounts of body solutions can be collected. Analytes may include endogenous molecules (e.g. neurotransmitter, hormones, glucose, etc.) to assess their biochemical functions in the body, or exogenous compounds (e.g. pharmaceuticals) to determine their distribution within the body. The micro dialysis technique may include the insertion of a small microdialysis catheter (also referred to as "micro dialysis probe" or "micro dialysis element") into the tissue of interest. The microdialysis probe is designed to mimic a blood capillary and consists of a shaft with a semipermeable hollow fiber membrane at its tip, which is connected to inlet and outlet tubing. The probe is continuously perfused with an aqueous solution (perfusate) that may especially closely resemble the (ionic) composition of the surrounding tissue fluid, and especially at a low flow rate of e.g. approximately 0.1-5µL/min. Once inserted into the tissue or (body) fluid of interest, small solutes can cross the semipermeable membrane by (passive) diffusion. Optionally, one may include one or more of albumin and cyclodextrine in perfusion medium (perfusate), especially to decrease non specific binding of sampled analytes.

The direction of the analyte flow is determined by the respective concentration gradient and allows the usage of microdialysis probes as sampling as well as delivery tools. The solution leaving the probe (dialysate) is e.g. collected at certain time intervals for analysis or may be measured continuously. To this end, e.g. the herein described sensing system may be applied. Hence, the biosensor may especially be used to sample analytes and thus provide samples, that may (temporarily) be stored (for later analysis) or directly be measured with the analysis unit.

The object of the present invention is achieved with a device according to the independent claim 1.

Especially, the invention provides a biosensor for at least partial insertion in a fetal scalp, for monitoring an analyte in the body of the fetus, wherein the biosensor comprises a needle and a microdialysis element for sampling the analyte. Alternatively, the biosensor may be used to release a material, such as a pharmaceutical. Hence, in a specific embodiment the membrane may be configured to allow passage of a pharmaceutical or nutrient. Hence, the biosensor or the sensing system may also be used for delivery of a pharmaceutical or nutrient (or neutraceutical) to a fetus.

For instance, in an embodiment the microdialysis element is integrated in the needle; especially the part comprising the membrane may be integrated in the needle.

In yet another embodiment, the biosensor comprises an electrode. Especially, the microdialysis element is integrated in the electrode.

The electrode, optionally comprising the microdialysis element, may especially have a single or double helix configuration.

In yet a further embodiment, the needle comprises the electrode and the microdialysis element.

In a specific embodiment, the microdialysis element comprises a membrane especially having a cut-off selected from the range of 2-400 kDa, such as 4-300 kDa, more especially 5-200 kDa, like 10-200 kDa.

Further, the invention provides a sensing system comprising the biosensor as defined herein, configured to flow a liquid through the microdialysis element. Especially, the sensing system includes an analysis unit, configured to sense an analyte in the liquid returning from the microdialysis element. Especially, the biosensor and/or sensing system are further configured to measure with the electrode one or more of CTG and STAN. The sensing system may especially be configured for continuously monitoring or configured for periodically monitoring an analyte in the body of the fetus. In a specific embodiment the sensing system is configured to sense lactate as analyte. Hence, especially the membrane may be configured to allow passage of the analyte(s) to be sampled and/or to be analyzed. In a further specific embodiment the sensing system is configured to perform an ELISA analysis. In yet a further specific embodiment the sensing system is configured to perform an enzymatic (assay) analysis. In yet a further specific embodiment the sensing system is configured to perform chromatographic analysis. In yet a further specific embodiment the sensing system is configured to perform mass spectrometric analysis, especially MSMS analysis. Hence, the sensing system may include the biosensor functionally coupled to one or more of a system for enzymatic (assay) analysis, a chromatographic unit (chromatographic apparatus), a mass spectrometric unit, etc.. Optionally, offline collection can be used. Samples can be stored for analysis later in time. Hence, the sensing system may include the biosensor and one or more of a storage unit and an analysis unit, wherein the biosensor can especially functionally coupled to one or more of said storage unit and said analysis unit. In an embodiment, the sensing system may thus further comprise a storage unit, functionally coupled with the biosensor, and configured to store a sample sampled with the biosensor. Hence the analyte(s) may be sampled in the perfusate and the thus obtained product may be - optionally after processing - be stored as (sampled) sample (or may directly be measured).

In a specific embodiment, the biosensor comprises a hollow membrane with a hollow tube (especially a tube in tube configuration) arranged in part of the cavity enclosed by the membrane, wherein the sensing system is configured to flow dialysis liquid via the hollow tube into the hollow membrane, and wherein the sensing system is configured to retrieve dialysis liquid that flow along the membrane back to an analysis unit.

In a specific embodiment, the analysis unit comprises one or more of an ELISA, an enzymatic assay, a chromatographic unit and a mass spectrometric unit.

Further, especially the invention provides the use of the biosensor or the sensing system according as defined herein, for sensing lactate and/or one or more other analytes in the scalp of a baby during giving birth.

Further, the invention provides the use of the biosensor or the sensing system as defined herein for sensing neurotransmitters in tissue of an animal.

The term "substantially" herein, such as in "substantially all light" or in "substantially consists", will be understood by the person skilled in the art. The term "substantially" may also include embodiments with "entirely", "completely", "all", etc. Hence, in embodiments the adjective substantially may also be removed. Where applicable, the term "substantially" may also relate to 90% or higher, such as 95% or higher, especially 99% or higher, even more especially 99.5% or higher, including 100%. The term "comprise" includes also embodiments wherein the term "comprises" means "consists of'. The term "and/or" especially relates to one or more of the items mentioned before and after "and/or". For instance, a phrase "item 1 and/or item 2" and similar phrases may relate to one or more of item 1 and item 2. The term "comprising" may in an embodiment refer to "consisting of' but may in another embodiment also refer to "containing at least the defined species and optionally one or more other species".

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The devices herein are amongst others described during operation. As will be clear to the person skilled in the art, the invention is not limited to methods of operation or devices in operation.

In the claims, any reference signs placed between parentheses shall not be construed as limiting the claim. Use of the verb "to comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

The various aspects discussed in this patent can be combined in order to provide additional advantages. Furthermore, some of the features can form the basis for one or more divisional applications.

### BRIEF DESCRIPTION OF THE DRAWINGS

Embodiments of the invention will now be described, by way of example only, with reference to the accompanying schematic drawings in which corresponding reference symbols indicate corresponding parts, and in which:
Fig.1a-2c schematically depict some aspects of the biosensor; and
Figs. 3a-4c show some results.

The schematic drawings are not necessarily on scale.

### DETAILED DESCRIPTION OF THE EMBODIMENTS

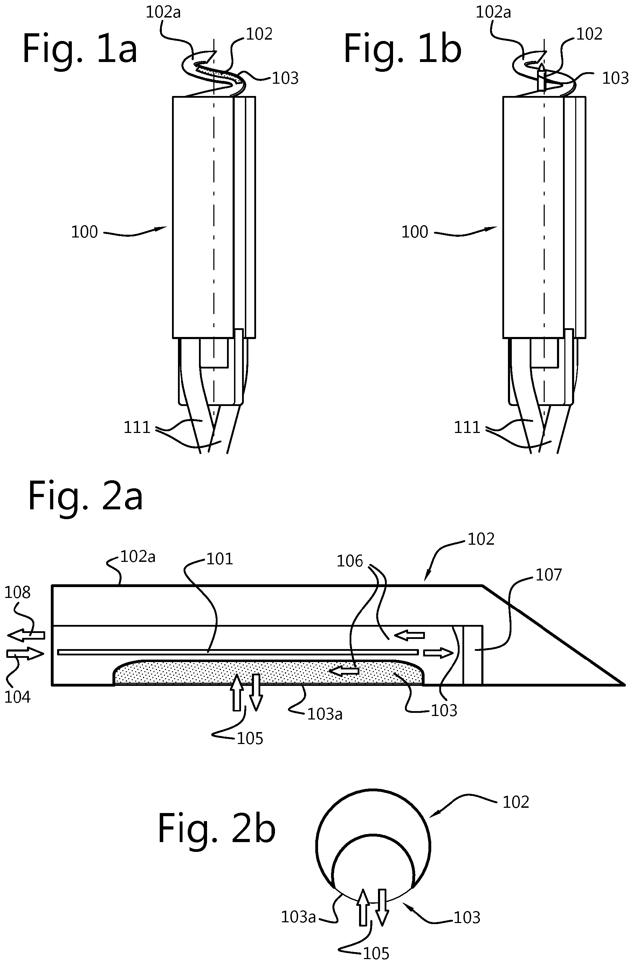

For adequate monitoring of e.g. lactate and/or other analytes, the microdialysis membrane needs to be inserted in the fetal scalp. To this end the microdialysis membrane can either be incorporated into (one of) the recording electrode(s) (fig 1a. Left) or can be incorporated into a scalp penetrating needle, that is located adjacent to the electrode(s), such as in the middle of a (helix) electrode (fig 1b Right). In the version where the microdialysis membrane is incorporated in a scalp penetrating needle, the needle will penetrate the scalp when the electrodes are screwed in. Figs. 1a-1b schematically depict embodiments of a (regular) fetal scalp electrode, indicated with reference 102, like a fetal scalp electrode configured for CTG analysis and/or STAN analysis, wherein the micro dialysis probe (also indicated herein as dialysis probe), indicated with reference 103, is indicated. Reference 100 indicates the biosensor.

A fetal scalp electrode (FSE) may especially monitor the fetal heart rate (FHR) directly and continuously during the intrapartum period. They accurately measure beat-to-beat variability and display the fetal electrocardiogram (fECG) waveform. The fetal scalp electrode allows electronic fetal monitoring (EFM). The electrode according to the invention includes a single helix configuration (see Figs. 1a/1b), or optionally a double helix configuration. Internal fetal monitoring especially involves placing an electrode directly on the fetal scalp through the cervix. This test is performed to evaluate fetal heart rate and variability between beats, especially in relation to the uterine contractions of labor. Internal monitoring differs from external monitoring. Instead of both leads being strapped to the outside of the woman's body, the Doppler lead is replaced by a smaller lead that is placed inside the woman's vagina and attached to the head of the baby. The internal lead is called a 'fetal scalp electrode' (or FSE).

It is especially only used to monitor the baby's heart rate during labour, usually if external monitoring is not being reliable (but sometimes if the caregiver prefers internal to external monitoring). A fetal scalp 'clip', or electrode, may be a small, circular, corkscrew-shaped needle attached to a coated wire. The clip may be covered with a long, protective, flexible, plastic covering and guided up through the mother's vagina by the caregiver doing an internal examination. This procedure should not be any more uncomfortable than a normal vaginal examination. The waters need to be broken to attach an FSE to the baby's head. If they are not already broken, this will need to be done to allow the electrode to be attached. The needle is gently rotated into the skin on the baby's scalp (or bottom if the baby is breech). The internal electrode monitors the baby's heart rate more accurately than an external Doppler. Once the clip is attached, the plastic cover is removed, leaving just the wire. The fetal scalp clip may have two colored wires attached. The wires are connected to the lead with a small conducting device (about the size of a match-box), strapped to the woman's thigh. The conducting device has conducting gel applied to it (like the gel used in ultrasounds). The lead is then plugged into the monitor to produce the readouts.

The electrode, especially for fetal monitoring or like application, may comprise an electrically conductive electrode lead having means at one end thereof to transmit a signal to a recording or related means, said lead being provided at its opposite end with a reversely reentrant, sharpened and barbed formation to embed in a skin area of a fetus, a second electrode lead generally paralleling said first lead and also provided with means at an end thereof to transmit a signal to said recording means, and a sheath encircling said leads and exposing said opposite end of said first-named lead.

In such electrode(s) the microdialysis element may be incorporated, such as in a recording electrode or in a separate needle (or both)(see also above).

The dialysis membrane, indicated with reference 103a which is comprised by the biosensor 100, typically has a molecular weight cut-off of 5KDa to 200 KDa. The membrane may be closed off on one side, e.g. with glue. By inserting a (thin) hollow tube 101, such as a fused silica tube, into the membrane, ringer's solution or saline can be perfused through the inner side of the membrane. The open surface of the membrane 103a will typically be (over a length of) 1-5 mm, which is enough to sample recover lactate and/or other analytes from the scalp for quantification (figs 2a-2b). This application can also be used in other target tissues, like regular skin or bone.

Hence, a (dialysis) liquid (herein also indicated as perfusate) is flowed in the internal of the microdialysis element along the dialysis membrane, thereby entraining (bio)molecules at the other side of the membrane when the membrane is in contact with body tissue and/or a body fluid.

Figs 1a-1b. Fetal scalp electrode for CTG and STAN analysis, in which a microdialysis membrane is incorporated. The dotter area in fig. 1a marks the position where the membrane is in contact with the extracellular fluid (during use). Left picture 1a shows the membrane incorporated into the CTG/STAN electrode. Right picture 1b shows an additional skin penetrating needle type micro dialysis probe, in which the membrane is incorporated. Hence, in both embodiments the micro dialysis probe is incorporated in the biosensor for CTG and/or STAN, herein also indicated as (regular) fetal scalp electrode. However, in the former (1a) the micro dialysis probe is integrated in the actual electrode(s) 102, whereas in the latter (1b) the micro dialysis probe is arranged adjacent to the actual electrode(s) 102 for CTG and/or STAN analysis, such as within the helix shaped electrode 102.

Fig 2a depicts an embodiment and some aspects of the dialysis procedure in both configurations of fig. 1 or other configurations. Here, Fig 2a especially shows a configuration of dialysis membranes incorporated in CTG/STAN electrode 102, though a similar configuration may also be used for the separate probe as depicted in fig. 1b. Reference 102a indicates a surface of the electrode 102. Dialysis liquid is transported to tube 104, which is arranged within the micro dialysis probe 103. The probe comprises membrane 103a. The dialysis fluid will leave the tube 104 and flow back, along the membrane 103a. The dialysis liquid may be forced back by a closure 107 at an end of the micro dialysis probe, such as a glue stop. While flowing along the membrane 103a, exchange 105 of (small) molecules will occur between the dialysis liquid and external fluid. Fluid can be collected when leaving 108 the dialysis probe 103. An opposite flow of the fluid within the dialysis probe 103 is also possible. Fig. 2b schematically depicts a cross-section of fig. 2a at the membrane 103a. Referring to figs. 2a-2b, part of the electrode surface 102a is open, such as over a length of 1-5 mm, such that the membrane 103a can be exposed to body tissue and body fluid. Fluid that is collected downstream from the membrane (see reference 108) may be analyzed as above.

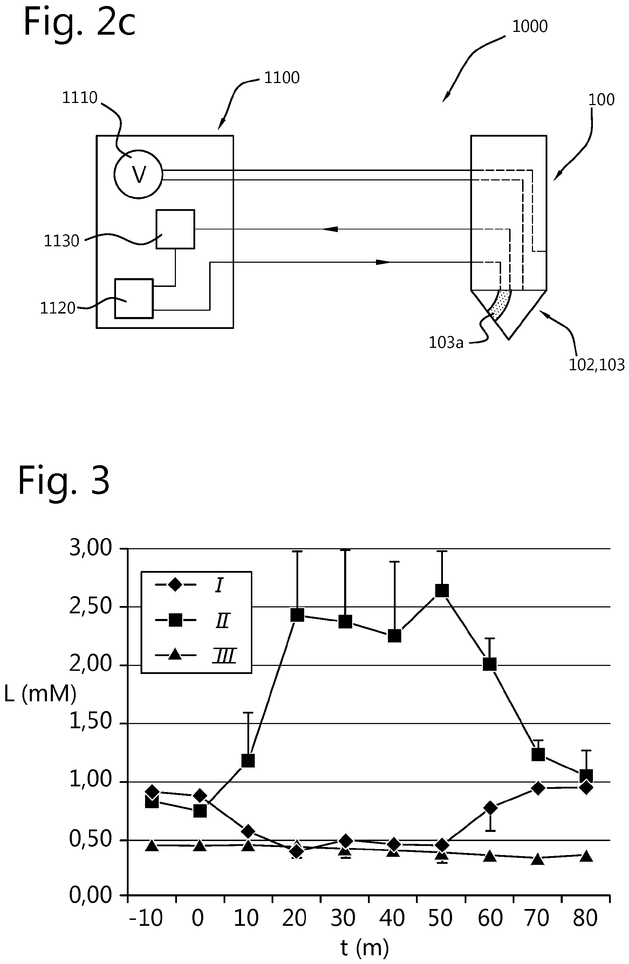

Fig. 2c schematically depicts a system 1000 with a biosensor 100 functionally coupled to an analysis unit 1100. Here, the analysis unit 1100 is optionally (also) configured to measure e.g. a CTG signal, using e.g. a voltage source or electro physiological measuring device 1110. Further, a dialysis fluid source 120 is functionally coupled with the biosensor 100 to provide the fluid, which returns to the analysis unit to be analyzed with a dialysis fluid analysis unit, comprised by the analysis unit 1100. Further, the term "analysis unit" may also refer to a plurality of such units, which may especially be functionally coupled. For instance, the analysis unit may include a chromatographic unit and a mass spectrometric unit. Hence, optionally the system may be configured to sample with the biosensor and to measure CTG and/or STAN.

Note that the unit 1100 may optionally also be configured to provide a nutrient and/or pharmaceutical. In such instance the unit 1100 may be defined as delivery unit. Further the unit 1100 is not necessarily configured to sense also via the electrode (functionality). For instance, the unit may be configured to (1) sense the analyte via the biosensor, and/or (2) to provide a nutrient and/or pharmaceutical via the biosensor, and optionally also to measure CTG and/or STAN. Instead of nutrient and/or pharmaceutical, also one or more of a contrast liquid or radioactive isotope, etc. may be included in the dialysis liquid.

Note however that the same principle may be used to release active components, like a pharmaceutical or a nutrient. Even, during application both functions may be applied, i.e. monitoring by e.g. lactate sensing as well as releasing a pharmaceutical. Hence, the biosensor and/or sensing system may also be used for delivery of a pharmaceutical or nutrient to a fetus.

### Experimental

Adult male wild type wistar rats were used for the experiments (500 g). Animals were anaesthetized using isoflurane (oxygen 0.8 1/min, nitrogen 0 l/min). After shaving, microdialysis probes were inserted in the skin of the back of the animal and fixated with a suture. A pulse oximeter was clamped on the tail of the animal to monitor oxygenation and heart rate. A jugular vena cannula was inserted to draw blood samples (100 microl each), for analysis of lactate and saturation (Abbott analyzer).

Dialysis was performed using sterile saline solution (dialysis liquid) at 1.5 microl/min. Samples were collected in 300 microl polypropylene vials, for lactate analysis. After stabilization of 15 min, sampling was started.

### Analysis

Lactate, pH and saturation in blood samples was analyzed using a handheld Abbott analyzer. Lactate from dialysis samples was quantified with an enzymatic assay in a 96 well plate (reagents CMA).

### Results

### Peripheral Lactate

Upon decreasing of oxygenation (t=0 min) using increased nitrogen (0.8 1/min) and decreased oxygen (0.051/min), a saturation could be reduced to below 40% for an hour. Blood lactate levels simultaneously increased 2.5 fold (Fig 3.).Upon restoration of saturation using 0.8 1/min oxygen, lactate levels returned to baseline levels rapidly. Blood pH, slowly decreased over the course of the experiment, and did not return to baseline for the 20 min when the animal was at restored saturation. Fig. 3 shows on the x-axis the time in minutes and on the y-axis lactate (L) in mM. Curve I (closed diamonds) indicates the saturation, curve II (closed squares) indicates lactate, and curve III (closed triangles) indicates the pH-7. 100% oxygenation is indicated with 1. The pH is the pH-7; i.e. the start pH is thus at 7.5.

### Microdialysis of lactate

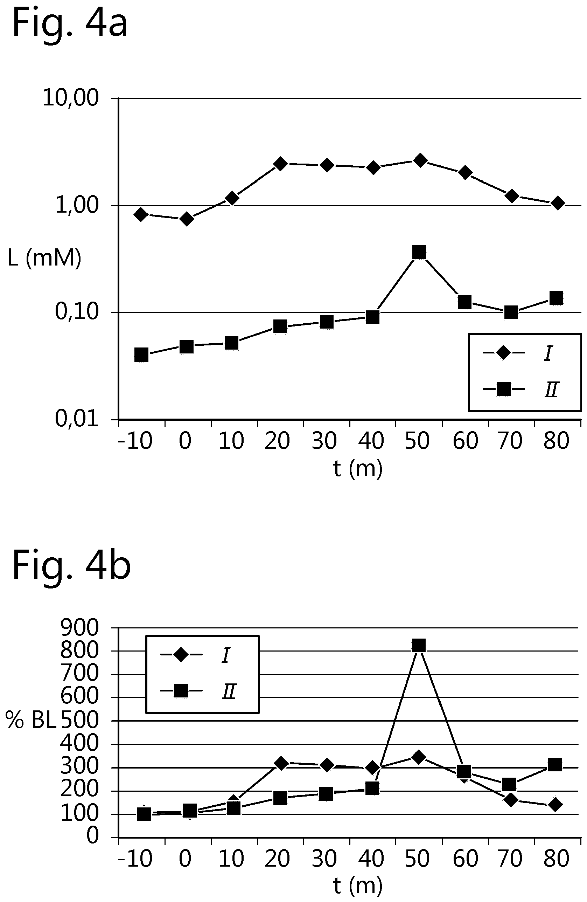

Figure 4a depicts the lactate levels as measured in dialysate and blood. The upper panel shows the absolute values of lactate from blood and dialysates (y- axis Lactate (L) in mM; and x-axis time in minutes). With a recovery of the dialysis membrane for lactate (II) of about 5%, levels corrected for recovery match blood lactate levels (I). Dialysis lactate levels increased at the same time that blood lactate levels increased, indicating that microdialysis can be used as alternative for blood lactate analysis. The lower panel 4b shows the effects represented as % of basal level (BL) (y- axis % of basal level (BL); and x-axis time in minutes). The high peak at about 50 minutes is an artefact.

### Recovery experiment

Recovery was performed by inserting a CTG electrode with integrated microdialysis membrane in a stirred saline solution containing 10 mM of lactate. A 200 µm outer diameter microdialysis membrane with 18 kDa cutoff was applied.

| Dialysis flow | Relative recovery (mean ± sem) |
|---|---|
| 1.5 microliter per minute | 22.74 ± 2.27 % (n=4)⁽¹⁾ |
| 5 microliter per minute | 8.90 ± 0.64 % (n=2) |

| | |
|---|---|
| ⁽¹⁾ Relative to 10 mM external lactate concentration. Hence, with 22% one measures 2.2 mM when using the biosensor in the 10 mM lactate bath. | |

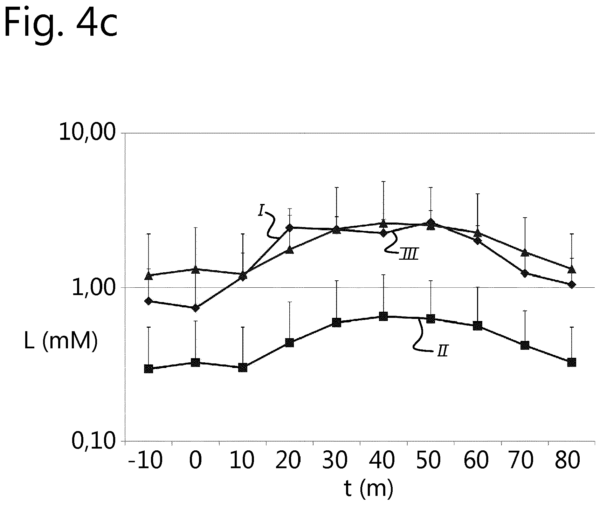

The data are also shown in Fig. 4c, with I indicating the blood lactate level, II indicating the concentration in the actual microdialysis fluid, and III indicating the corrected concentration of lactate in dialysis fluid after correction for recovery 22.74 %.

Hence, the invention provides a biosensor according to claim 1 The invention also provides a sensing system comprising the biosensor according to any one of the preceding system, configured to flow a liquid through the micro dialysis element. The sensing system may include one or more units configured to analyze the liquid that flows back from the biosensor. Further, the sensing system may include a unit to flow the dialysis liquid through the biosensor. For instance, the biosensor may comprise a hollow membrane with a hollow tube (especially a tube in tube configuration) arranged in part of the cavity enclosed by the membrane (see also Figs. 2a-2b), wherein the sensing system is configured to flow dialysis liquid via the hollow tube into the hollow membrane, and wherein the sensing system is configured to retrieve dialysis liquid that flow along the membrane back to an analysis unit. The invention also provides the use of the biosensor as defined herein or the sensing system according as defined herein, for sensing lactate in the scalp of a baby during giving birth. Especially, the fetal scalp is of a human fetus. The invention is defined in the accompanying claims.

## Claims

1. A biosensor for at least partial insertion in a fetal scalp, for monitoring an analyte in the body of the fetus, wherein the biosensor comprises a needle, an electrode (102) and a microdialysis element (103) for sampling the analyte, wherein the electrode has a single or double helix configuration, and wherein:
the microdialysis element is integrated in the needle; or
the microdialysis element is integrated in the electrode.

2. The biosensor according to claim 1, wherein when the microdialysis element is integrated in the needle, the needle comprises the electrode and the microdialysis element.

3. The biosensor according to any one of the preceding claims, wherein the microdialysis element comprises a membrane having a cut-off selected from the range of 5-200 kDa.

4. A sensing system comprising the biosensor according to any one of the preceding claims, configured to flow a liquid through the microdialysis element.

5. The sensing system according to claim 4, wherein the sensing system includes an analysis unit, configured to sense an analyte in the liquid returning from the microdialysis element, wherein the sensing system is configured for continuously monitoring or configured for periodically monitoring an analyte in the body of the fetus.

6. The sensing system according to any one of claims 4-5, further configured to measure with the electrode one or more of CTG and STAN.

7. The sensing system according to any one of claims 4-6, wherein the sensing system is configured to sense lactate as analyte.

8. The sensing system according to any one of claims 4-7, wherein the sensing system is configured to perform one or more analyses selected from the group consisting of an ELISA analysis, an enzymatic assay analysis, chromatographic analysis, and mass spectrometric analysis, especially MSMS analysis.

9. The sensing system according to any one of claims 4-8, wherein the biosensor comprises a hollow membrane with a hollow tube, especially a tube in tube configuration, arranged in part of the cavity enclosed by the membrane, wherein the sensing system is configured to flow dialysis liquid via the hollow tube into the hollow membrane, and wherein the sensing system is configured to retrieve dialysis liquid that flow along the membrane back to an analysis unit.

10. The sensing system according to claim 9, wherein the analysis unit comprises one or more of an ELISA, an enzymatic assay, a chromatographic unit and a mass spectrometric unit.

11. The sensing system according to any one of claims 4-10, further comprising a storage unit, functionally coupled with the biosensor, and configured to store a sample sampled with the biosensor.

12. Use of a previously inserted biosensor according to any one of claims 1-3 or a sensing system comprising a previously inserted biosensor according to any one of claims 4-11, for (i) sensing lactate and/or one or more other analytes in the scalp of a baby during giving birth.

13. Use of a previously inserted biosensor according to any one of claims 1-3 or a sensing system comprising a previously inserted biosensor according to any one of claim 4-11, for sensing neurotransmitters in tissue of an animal.

## Patentansprüche

1. Biosensor zum mindestens teilweisen Einführen in eine Kopfschwarte eines Fötus zum Monitoring eines Analyten im Körper des Fötus, wobei der Biosensor eine Nadel, eine Elektrode (102) und ein Mikrodialyseelement (103) zur Abtastung des Analyten umfasst, wobei die Elektrode eine Einzel- oder Doppelhelixkonfiguration hat, und wobei:
das Mikrodialyseelement in die Nadel integriert ist; oder
das Mikrodialyseelement in die Elektrode integriert ist.

2. Biosensor nach Anspruch 1, wobei die Nadel, wenn das Mikrodialyseelement in die Nadel integriert ist, die Elektrode und das Mikrodialyseelement umfasst.

3. Biosensor nach einem der vorhergehenden Ansprüche, wobei das Mikroanalyseelement eine Membran mit einer Abschneidung (Cutoff) ausgewählt aus dem Bereich von 5 bis 200 kDa umfasst.

4. Abfühlsystem, das den Biosensor gemäß einem der vorhergehenden Ansprüche umfasst und ausgestaltet ist, um eine Flüssigkeit durch das Mikrodialyseelement fließen zu lassen.

5. Abfühlsystem nach Anspruch 4, wobei das Abfühlsystem eine Analyseeinheit einschließt, die ausgestaltet ist, um einen Analyten in der Flüssigkeit abzufühlen, die von dem Mikrodialyseelement zurückläuft, wobei das Abfühlsystem zum kontinuierlichen Monitoring oder zum periodischen Monitoring eines Analyten im Körper des Fötus ausgestaltet ist.

6. Abfühlsystem nach einem der Ansprüche 4 bis 5, das des Weiteren ausgestaltet ist, um mit der Elektrode ein oder mehrere von CTG und STAN zu messen.

7. Abfühlsystem nach einem der Ansprüche 4 bis 6, wobei das Abfühlsystem ausgestaltet ist, um Lactat als Analyt abzufühlen.

8. Abfühlsystem nach einem der Ansprüche 4 bis 7, wobei das Abfühlsystem ausgestaltet ist, um eine oder mehrere Analysen ausgewählt aus der Gruppe bestehend aus einer ELISA-Analyse, einer Analyse mit enzymatischem Assay, einer chromatographischen Analyse und einer massenspektrometrischen Analyse, insbesondere MSMS-Analyse, durchzuführen.

9. Abfühlsystem nach einem der Ansprüche 4 bis 8, wobei der Biosensor eine Hohlmembran mit einem hohlen Rohr umfasst, insbesondere eine Rohr-in-RohrKonfiguration, die in einem Teil des von der Membran umschlossenen Hohlraums angeordnet ist, wobei das Abfühlsystem ausgestaltet ist, um Dialyseflüssigkeit über das hohle Rohr in die Hohlmembran fließen zu lassen, und wobei das Abfühlsystem ausgestaltet ist, um Dialyseflüssigkeit zurückzubekommen, die an der Membran entlang zurück zu einer Analyseeinheit fließt.

10. Abfühlsystem nach Anspruch 9, wobei die Analyseeinheit ein oder mehrere von einem ELISA, einem enzymatischen Assay, einer chromatographischen Einheit und einer massenspektrometrischen Einheit umfasst.

11. Abfühlsystem nach einem der Ansprüche 4 bis 10, des Weiteren umfassend eine Speichereinheit, die funktional mit dem Biosensor gekoppelt ist und ausgestaltet ist, um eine mit dem Biosensor abgetastete Probe zu speichern.

12. Verwendung eines zuvor eingeführten Biosensors gemäß einem der Ansprüche 1 bis 3 oder eines Abfühlsystems, das einen zuvor eingeführten Biosensor umfasst, gemäß einem der Ansprüche 4 bis 11, zum (i) Abfühlen von Lactat und/oder einem oder mehreren Analyten in der Kopfhaut eines Babys während des Geburtsvorgangs.

13. Verwendung eines zuvor eingeführten Biosensors gemäß einem der Ansprüche 1 bis 3 oder eines Abfühlsystems, das einen zuvor eingeführten Biosensor umfasst, gemäß einem der Ansprüche 4 bis 11, zum Abfühlen von Neurotransmittern in Gewebe eines Tieres.

## Revendications

1. Biocapteur destiné à être inséré au moins partiellement dans un cuir chevelu fœtal, pour surveiller un analyte dans le corps du fœtus, le biocapteur comprenant une aiguille, une électrode (102) et un élément de microdialyse (103) pour échantillonner l'analyte, l'électrode ayant une configuration à simple ou double hélice, et
l'élément de microdialyse étant intégré dans l'aiguille ; ou
l'élément de microdialyse étant intégré dans l'électrode.

2. Biocapteur selon la revendication 1, lorsque l'élément de microdialyse est intégré dans l'aiguille, l'aiguille comprenant l'électrode et l'élément de microdialyse.

3. Biocapteur selon l'une quelconque des revendications précédentes, l'élément de microdialyse comprenant une membrane ayant une coupure choisie dans la gamme de 5-200 kDa.

4. Système de détection comprenant le biocapteur selon l'une quelconque des revendications précédentes, configuré pour faire circuler un liquide à travers l'élément de microdialyse.

5. Système de détection selon la revendication 4,
le système de détection comprenant une unité d'analyse, configurée pour détecter un analyte dans le liquide revenant de l'élément de microdialyse, le système de détection étant configuré pour surveiller en continu ou configuré pour surveiller périodiquement un analyte dans le corps du fœtus.

6. Système de détection selon l'une quelconque des revendications 4 et 5, configuré en outre pour mesurer avec l'électrode un ou plusieurs des CTG et STAN.

7. Système de détection selon l'une quelconque des revendications 4 à 6, le système de détection étant configuré pour détecter le lactate comme analyte.

8. Système de détection selon l'une quelconque des revendications 4 à 7, le système de détection étant configuré pour réaliser une ou plusieurs analyses choisies dans le groupe constitué d'une analyse ELISA, d'une analyse par dosage enzymatique, d'une analyse chromatographique et d'une analyse par spectrométrie de masse, en particulier une analyse MSMS.

9. Système de détection selon l'une quelconque des revendications 4 à 8, le biocapteur comprenant une membrane creuse avec un tube creux, en particulier un tube dans une configuration tubulaire, agencé dans une partie de la cavité entourée par la membrane, le système de détection étant configuré pour faire circuler du liquide de dialyse via le tube creux dans la membrane creuse, et le système de détection étant configuré pour récupérer le liquide de dialyse qui circule le long de la membrane pour le renvoyer vers une unité d'analyse.

10. Système de détection selon la revendication 9, l'unité d'analyse comprenant un ou plusieurs éléments parmi un ELISA, un dosage enzymatique, une unité chromatographique et une unité de spectrométrie de masse.

11. Système de détection selon l'une quelconque des revendications 4 à 10, comprenant en outre une unité de stockage, couplée fonctionnellement au biocapteur, et configurée pour stocker un échantillon échantillonné avec le biocapteur.

12. Utilisation d'un biocapteur préalablement inséré selon l'une quelconque des revendications 1 à 3 ou d'un système de détection comprenant un biocapteur préalablement inséré selon l'une quelconque des revendications 4 à 11, pour (i) détecter le lactate et/ou un ou plusieurs autres analytes dans le cuir chevelu d'un bébé pendant l'accouchement.

13. Utilisation d'un biocapteur précédemment inséré selon l'une quelconque des revendications 1 à 3 ou d'un système de détection comprenant un biocapteur précédemment inséré selon l'une quelconque des revendications 4 à 11, pour détecter des neurotransmetteurs dans le tissu d'un animal.
